# EUROPEAN PATENT APPLICATION

(11) **EP 2 169 573 A2**
(43) Date of publication of application: **31.03.2010**
(21) Application number: 09171005.3
(22) Date of filing: 22.09.2009
(51) Int. Cl.: G06F 19/00

(54) **Predicting rare events using principal component analysis and partial least squares**

(30) Priority: 25.09.2008 US 284929
(71) Applicant: AIR PRODUCTS AND CHEMICALS, INC., Allentown, PA 18195-1501 (US)
(72) Inventor: Mehta, Sanjay, Alburtis PA 18011 (US); Neogi, Debashis, Emmaus PA 18049 (US)
(74) Representative: Stones, James Alexander

(57) **Abstract**

Systems and methods are provided for predicting rare events, such as hospitalization events. Data related to health and/or healthcare may be compiled from a number of sources and used to construct a predictive model. The predictive model employ Principal Component Analysis (PCA) and Partial Least Squares (PLS). The data may be arranged in a timeline, and formatted in such a way as to provide discrete temporal "batches". This arrangement may facilitate the PCA and PLS decomposition of the data into predictive models. These models may then be applied to an individual's data, to create a prediction of healthcare related events.

## Description

### BACKGROUND OF THE INVENTION

Predicting rare events, like Hospitalization for a given patient suffering from chronic disease, is difficult to model using traditional techniques. Most traditional data-mining methodologies like Neural Networks and Logistical Regression, do not account for longitudinal time effects for each patient. Additionally, correlations are built between the target variable and the original set of predictor variables and tends to treat them independently. Whereas, in reality, many of the predictor variables are highly correlated.

### BRIEF SUMMARY OF THE INVENTION

Example embodiments of the present invention relate to predicting rare event outcomes using Principal Component Analysis (PCA) and Partial Least Squares (PLS). One example of a rare event that may be predicted by example embodiments of the present invention is a hospitalization event within a certain time period for a particular person. Hospitalization events are traumatic and expensive, requiring accurate predictions for the benefit of the patient, the patient's doctor/caregiver, and insurance companies who insure the patient.

PCA and PLS techniques capture correlations among various predictor variables. These methods also explain the variability of a system in terms of a few principal components (e.g., a composite variable created based on a linear combination of predictor variables). This re-parameterization is unique in the sense that it keeps the information intact for all the original variables. PCA techniques are powerful and efficient for building a reduced order model for categorical and continuous predictor variables. For example, a PCA model based on patient historical data can be used to create a decision flag indicating whether a patient requires hospitalization. PLS helps to explain the variability in a continuous target/response variable in terms of predictor variables. An example target variable may be the length of a hospital stay or the cost associated with a hospitalization or time to hospitalization.

### BRIEF DESCRIPTION OF SEVERAL VIEWS OF THE DRAWINGS

Fig. 1 illustrates an example procedure, according to an example embodiment of the present invention.
Fig. 2 illustrates an example data layout, according to an example embodiment of the present invention.
Fig. 3 illustrates an example matrix layout, according to an example embodiment of the present invention.
Fig. 4 illustrates an example system, according to an example embodiment of the present invention.

### DETAILED DESCRIPTION OF THE INVENTION

Example embodiments of the present invention relate to predicting rare event outcomes using Principal Component Analysis (PCA) and Partial Least Squares (PLS). One example of a rare event that may be predicted by example embodiments of the present invention is a hospitalization event within a certain time period for a particular person. Example embodiments of the present invention may generally comprise four steps. First, the example embodiment may collect historical data, including non-target events and target events. Next, the example embodiment may create a model based on this historical data. Third, the example embodiment may apply the model to an individual's data. Finally, the example embodiment may create a prediction based on the model applied to that particular data. Additionally, the example embodiment may use PCA and PLS to create the predictive model.

Data used in the predictor model may be pulled from a number of sources, and the types of data will depend on the event to be predicted. One example may be hospitalization events; meaning, based on data and the sequence of events occurring with respect to a specific person, predicting the likelihood that that person will require hospitalization in any given timeframe. In the example of predicting hospitalization events, relevant data may include: personal data about the patient's background and health data about the patient's medical history, etc. Examples may include: date of birth, height (after a certain age), ethnicity, gender, family history, geography (e.g., place where the patient lives), family size including marital status, career field, education level, medical charts, medical records, medical device data, lab data, weight gain/loss, prescription claims, insurance claims, physical activity levels, climate changes of patient-location, and any number of other medical or health related metrics, or any number of other pieces of data. Data may be pulled from any number of sources, include patient questionnaires, text records (e.g., text data mining of narrative records), data storage of medial devices (e.g., data collected by a heart monitor), health databases, insurance claim databases, etc.

Data that is useful to the model in a native format may be directly imported into a prediction event database. Other data may need to be transformed into a useful state. Still other data may be stored with unnecessary components (e.g., data contained in a text narrative). In this latter situation, a text mining procedure may need to be implemented. Text mining and data mining are known in the art and several commercial products exist for this purpose. Alternatively, a proprietary procedure may be used to mine text for relevant event data. Data may be pulled from a number of sources and stored in a central modeling database. The modeling database may consist of one data repository in one location, more than one data repository in one location, or more than one data repository in more than one location.

Example embodiments of the present invention provide a powerful event modeler, being able to predict, for example, both when a hospitalization event will occur and how long it will last and/or how much it will cost. In example embodiments of the present invention, the time between regularly scheduled doctor visits or any other time stamps may be used to partition the patient history data into discrete time windows. The partitioning may be variable or uniform in length. In this respect, the modeling is similar to modeling a chemical plant failure. Chemical plants may be modeled based on "batches", with certain events occurring during a batch, to predict a plant failure. In terms of hospitalization events, periods of time between doctor visits where no hospitalization event occurred may be considered a "good" batch. Whereas periods of time between doctor visits where there was a hospitalization event may be considered a "bad" batch. Various other events and data may occur during the time intervals. Some events may be single events (e.g., experiencing an asthma attack), and other events may be continuous (e.g., weight or pacemaker data). An advantage to this example embodiment is that "lag" variables (e.g., no hospitalization event for some period of time) are inherently incorporated into the predictive model.

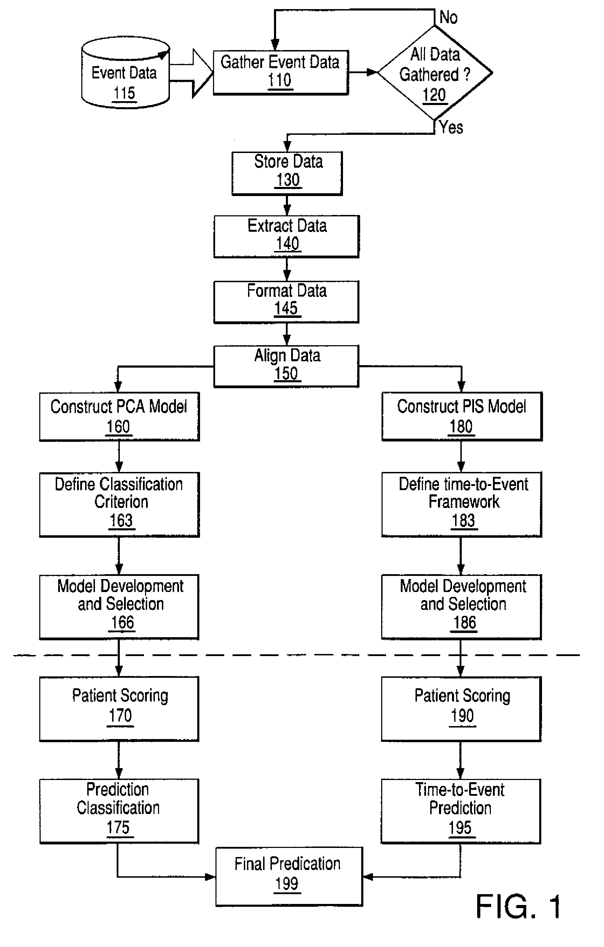

Fig. 1 illustrates one example procedure for collecting, preparing, and applying data in a PCA/PLS model. The example procedure illustrated in Fig. 1 will be discussed in terms of the patient/hospitalization example, but the example procedure could be applied to any event-based prediction model. At 110, the example procedure may gather event data. This could be any kind of data (e.g., the types of data listed above) and could be from any source. Some data may come from the patients themselves. Some data may come from devices associated with patients (e.g., a pacemaker, systems monitor, cellular telephone, etc.). Some data may come from medical databases or other database repositories. At 120, once all the data, from all the sources (e.g., 115), is gathered, the example procedure may store the data at 130, (e.g., in a database).

The data may next undergo one or more "data preparation" phases. For example, at 140, data may be extracted from various raw text formats using data-mining techniques. At 145, the data may be formatted. This may include transforming the data to conform to some standard or otherwise tagging relevant parts of the data. For example, diagnosis data may be formatted according to a standard coding scheme, such as an ICD notation (i.e., "International Classification of Diseases") (e.g., ICD-9). Next, at 150, the example procedure may align the data. This may include organizing the data according to time-stamps or some other indication of when the event occurred or data was initially collected. A temporal alignment may allow for temporal patterns to be observed in the data-sets. Any variety of other data preparation is also possible.

At this point in the example embodiment, the example procedure may construct two different models. These constructions may occur in parallel, as shown, or in any other order (e.g., a serial order). At 160, the example procedure may construct a PCA model. This may generally include constructing a matrix of the different responses, calculating a covariate matrix, and calculating the eigenvalue decomposition of the covariate matrix. Other PCA variations are possible, including other singular-value decompositions. At 163, the example procedure may define a classification criterion. Examples related to the example of hospitalizations, may include the length of the hospital stay, or the cost of the hospital stay. At 166, the example procedure may combine the matrix constructions and decompositions with the relevant event classification (e.g., cost of hospitalization) to construct a PCA prediction model. At this point, the example procedure may transition from "model building" based on historical records, to "model application" based on an individual's present data. At 170, the example procedure may apply an individual's data to the constructed model to create a patient score, or otherwise evaluate the patient data with respect to the model. At 175, the example procedure may create a prediction based on the classification criteria.

Concurrently with the PCA model, the example procedure may construct a PLS model at 180. At 183, the example method may define the time-to-event framework to be predicted. This may include several things, such as, assigning the event to be predicted (e.g., a hospitalization), and assigning the time frame for the event (e.g., within the next week or within the next month). At 186, the example procedure may construct a PLS model of the stored data to predict the relevant event outlined at 183. Similar to 170, at 190, the PLS model may be applied to a set of patient data to provide a score, or otherwise evaluate the data associated with a patient. At 195, the example procedure may produce a time to event prediction (e.g., the probability the patient will experience a hospitalization event in the next month). At the end of the example procedure, a final prediction may be produced, combining both discrete and continuous predictive results, (e.g., the probability of an event, and the probable length of the event).

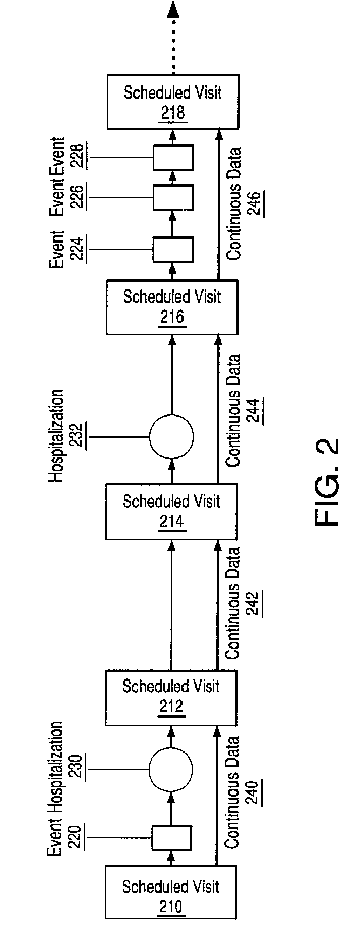

Data used in the PCA/PLS model may be best organized according to time, and partitioned into discrete chunks of time. In this way, during the data preparation phase of example embodiments, the data may be organized as illustrated by Fig. 2. As described above, the data may be laid out in a similar fashion as process data used to predict plant failures. In Fig. 2 the data may include discrete events (e.g., 220, 224, 226, and 228) and continuous data (e.g., 240, 242, 244, and 246). These sets of data may be the same or different than the other sets. For example, continuous data 240 may be recorded data from a pacemaker, whereas continuous data 242 may be just the continued use of the pacemaker, or a doctor may have added another monitoring device at scheduled visit 212. The data may be partitioned according to scheduled visits or any other time stamps (e.g., 210, 212, 214, 216, and 218). Then, any partition containing one or more of the target events, (e.g., Hospitalization 230 or 232) may be regarded as a "negative" outcome of varying degree, based on the other data present in the partition. Also, any partition with no target event may be regarded as a "positive" outcome of varying degree, based on the data present. Within each partition there are M time points where continuous and discrete data will be collected/interpolated. Each partition, whether "positive" or "negative" may be used to build the prediction model. Once the prediction model is constructed, it may be applied to a patient's data (e.g., the example data illustrated in Fig. 2). The model may then provide a probability of a future target event, such as the probability that this patient will experience a hospitalization event after scheduled visit 218.

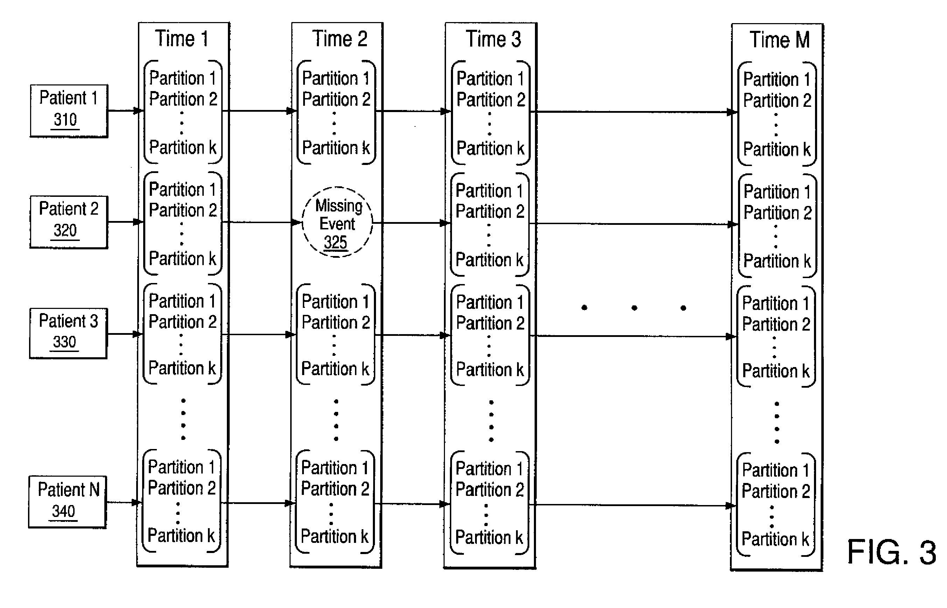

As PCA and PLS deal with the decomposition and manipulation of matrix data, the example embodiments of the present invention may need to organize the data in matrix form. Fig. 3 illustrates one example of this, where N patients with K partitions and each partition having data at M time points form an N*K by M matrix of historical data. In general K can have different values for different patients. Each vector (1 by K) in Fig. 3 may represent a time point for a particular patient (e.g., first blood pressure measurement after a given scheduled visit). From this matrix, a covariance matrix may be formed and eigenvalues/vectors may be calculated. The matrix of data does not have to be complete, but the more data present the better. In some instances an entire vector (1 by K) will be missing, such as missing data 325 (i.e., the second time slot for the second patient). Additionally, certain partition values within a data vector that are expected to be present may be missing. Each vector (e.g., partitions 1 to K) may have a different quantity of data and different data points. However, certain datasets may generally have one or more data types (e.g., patient weight) in every vector, but also have some exceptions where this expected value is missing. Missing data at the partition level or vector level is still useful in building a prediction model with example embodiments of the present invention.

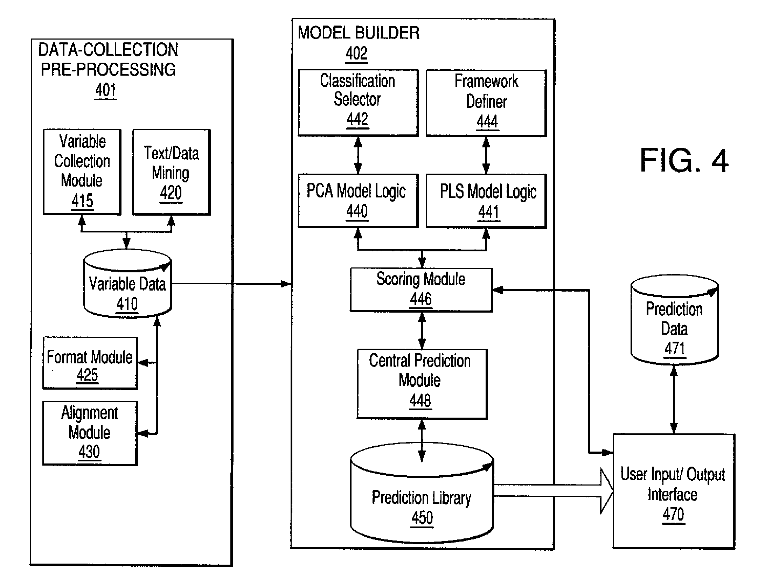

Fig. 4 illustrates an example system according to an example embodiment of the present invention. 401 may illustrate a data collection, preparation, and preprocessing component. This may include a data repository 410 for holding all of the variables used in the model constructing process. There may be a variable collection module 415 that may collect various data records from one or more sources. There may be a text and/or data mining module 420. This module may extract relevant information from textual narratives, journals, diaries, articles, etc. Once these modules (e.g., 415 and 420) collect the relevant data records, other modules may be used to adjust, standardize, and otherwise prepare the data to be organized in a decision tree. For example, a format module 425 may transform data into a recognized format or otherwise standardize the data. An alignment module 430 may organize the separate data records (each with one or more attributes) to line up based on some dimension (e.g., time).

Once the data has been collected, pre-processed, and otherwise prepared for modeling, the variable data may be imported, transmitted, or otherwise made accessible to a model building component 402. This component may be responsible for constructing the various matrices required for the PCA and/or PLS models. The component may contain construction logic 440 and 441, which may contain PCA and PLS logic respectively. There may be a classification selector 442 to select one or more criterion for the target event. There may be a framework definer 444, which may select the target event and/or define relevant parameters for the target event (e.g., a timeframe for the event to occur in). The scoring module 446 may receive a patient's data from the example system's user (e.g., data 471 from user input/output interface 470). This is only one example. Prediction data 471 may be a part of variable data 410, or stored anywhere else. The central prediction module 448 may combine the PCA and PLS predictions into a final probability. The outcome may be stored in a library (e.g., prediction library 450), and/or may be directly outputted to the user (e.g., 470). There may also be a user I/O interface 470 used to experiment, adjust, and otherwise administrate the example modeling system illustrated in Fig. 4. The example system of Fig. 4 may reside on one or more computer systems. These one or more systems may be connected to a network (e.g., the Internet). The one or more systems may have any number of computer components known in the computer art, such as processors, storage, RAM, cards, input/output devices, etc.

A hospitalization event was used in this description as an example, but is only one example of a rare event that may be predicted by models produced and run by example embodiments of the present invention. Any rare event and data associated with the rare event may be modeled and predicted using example embodiments of the present invention. Example embodiments may predict when a production factory goes offline. Events may include: downtime per each piece of equipment, error messages per each piece of equipment, production output, employee vacations, employee sick days, experience of employees, weather, time of year, power outages, or any number of other metrics related to factory production capacity. Factory data (e.g., records) may be proposed, measured, and assimilated into a model. The model may be used to compare known data about events at a factory. The outcome of that comparison may lead to the probability the factory goes offline. It may be appreciated that any rare event and set of related events may be used in conjunction with example embodiments of the present invention to predict the probability of that rare event occurring.

The various systems described herein may each include a computer-readable storage component for storing machine-readable instructions for performing the various processes as described and illustrated. The storage component may be any type of machine readable medium (i.e., one capable of being read by a machine) such as hard drive memory, flash memory, floppy disk memory, optically-encoded memory (e.g., a compact disk, DVD-ROM, DVD±R, CD-ROM, CD±R, holographic disk), a thermomechanical memory (e.g., scanning-probe-based data-storage), or any type of machine readable (computer readable) storing medium. Each computer system may also include addressable memory (e.g., random access memory, cache memory) to store data and/or sets of instructions that may be included within, or be generated by, the machine-readable instructions when they are executed by a processor on the respective platform. The methods and systems described herein may also be implemented as machine-readable instructions stored on or embodied in any of the above-described storage mechanisms. The various communications and operations described herein may be performed using any encrypted or unencrypted channel, and storage mechanisms described herein may use any storage and/or encryption mechanism.

Aspects and embodiments of the present invention include:
(a) A method, comprising:
   loading a plurality of data records;
   assigning an event to be predicted, wherein the event is related to the health or health-care of a person;
   constructing a prediction model, based at least in part on the plurality of data records, using at least one of the group including: Principal Component Analysis (PCA) and Partial Least Squares (PLS).
   The event is preferably a hospitalization.
   The method preferably further comprises preparing the plurality of data records. The preparing may include at least one of: data-mining, temporal alignment, and reformatting at least one record from the plurality of data records. The preparing may include a temporal alignment of the plurality of data records and organizing the plurality of data records into partitions, wherein each partition includes data records within a time period. The time period preferably includes the time between regularly scheduled visits to a healthcare provider.
   The prediction model is preferably constructed using both PCA and PLS.
   The method may further comprise:
   applying the model to data associated with an individual patient; and
   producing a prediction, based at least in part on the applying, for the individual patient.
(b) A system, comprising:
   a memory configured to store a plurality of data records;
   a processor configured to load a plurality of data records;
   the processor configured to assign an event to be predicted, wherein the event is related to the health or health-care of a person;
   the processor, in communication with the memory, configured to construct a prediction model, based at least in part on the plurality of data records, and configured to use at least one of the group including: Principal Component Analysis (PCA) and Partial Least Squares (PLS).
   The event is preferably a hospitalization.
   The system may further comprise the processor configured to prepare the plurality of data records. The preparing may includes at least one of: data-mining, temporal alignment, and reformatting at least one record from the plurality of data records. The preparing may includes a temporal alignment of the plurality of data records and organizing the plurality of data records into groups, wherein each group includes data records from a particular time period. The time period preferably includes the time between regularly scheduled visits to a healthcare provider.
   The prediction model is preferably constructed using both PCA and PLS.
   The system preferably further comprises:
   applying the model to data associated with an individual patient; and
   producing a prediction, based at least in part on the applying, for the individual patient.
(c) A computer-readable storage medium encoded with instructions configured to be executed by a processor, the instructions which, when executed by the processor, cause the performance of a method, comprising:
   loading a plurality of data records;
   assigning an event to be predicted, wherein the event is related to the health or health-care of a person;
   constructing a prediction model, based at least in part on the plurality of data records, using at least one of the group including: Principal Component Analysis (PCA) and Partial Least Squares (PLS).

Although the present invention has been described with reference to particular examples and embodiments, it is understood that the present invention is not limited to those examples and embodiments. The present invention as claimed therefore includes variations from the specific examples and embodiments described herein, as will be apparent to one of skill in the art.

## Claims

1. A method, comprising:
loading a plurality of data records;
assigning an event to be predicted, wherein the event is related to the health or health-care of a person;
constructing a prediction model, based at least in part on the plurality of data records, using at least one of the group including: Principal Component Analysis (PCA) and Partial Least Squares (PLS).

2. A method as claimed in claim 1, further comprising:
preparing the plurality of data records.

3. A method as claimed in claim 2, wherein the preparing includes at least one of:
data-mining, temporal alignment, and reformatting at least one record from the plurality of data records.

4. A method as claimed in claim 2, wherein the preparing includes a temporal alignment of the plurality of data records and organizing the plurality of data records into partitions, wherein each partition includes data records within a time period.

5. A method as claimed in claim 4, wherein the time period includes the time between regularly scheduled visits to a healthcare provider.

6. A method as claimed in any preceding claim, further comprising:
applying the model to data associated with an individual patient; and
producing a prediction, based at least in part on the applying, for the individual patient.

7. A system, comprising:
a memory configured to store a plurality of data records;
a processor configured to load a plurality of data records;
the processor configured to assign an event to be predicted, wherein the event is related to the health or health-care of a person;
the processor, in communication with the memory, configured to construct a prediction model, based at least in part on the plurality of data records, and configured to use at least one of the group including: Principal Component Analysis (PCA) and Partial Least Squares (PLS).

8. A method as claimed in any of claims 1 to 6 or a system as claimed in claim 7, wherein the event is a hospitalization.

9. A system as claimed in claim 7 or claim 8, further comprising:
the processor configured to prepare the plurality of data records.

10. A system as claimed in claim 9, wherein the preparing includes at least one of:
data-mining, temporal alignment, and reformatting at least one record from the plurality of data records.

11. A system as claimed in claim 9, wherein the preparing includes a temporal alignment of the plurality of data records and organizing the plurality of data records into groups, wherein each group includes data records from a particular time period.

12. A system as claimed in claim 11, wherein the time period includes the time between regularly scheduled visits to a healthcare provider.

13. A method as claimed in any of claims 1 to 6 and 8 or a system as claimed in any of claims 7 to 12, wherein the prediction model is constructed using both PCA and PLS.

14. A system as claimed in any of claims 7 to 13, further comprising:
applying the model to data associated with an individual patient; and
producing a prediction, based at least in part on the applying, for the individual patient.

15. A computer-readable storage medium encoded with instructions configured to be executed by a processor, the instructions which, when executed by the processor, cause the performance of a method, comprising:
loading a plurality of data records;
assigning an event to be predicted, wherein the event is related to the health or health-care of a person;
constructing a prediction model, based at least in part on the plurality of data records, using at least one of the group including: Principal Component Analysis (PCA) and Partial Least Squares (PLS).
